# EUROPEAN PATENT SPECIFICATION

(11) **EP 1 935 408 B1**
(45) Date of publication and mention of the grant of the patent: **01.09.2010**
(21) Application number: 06425838.7
(22) Date of filing: 15.12.2006
(51) Int. Cl.: A61K 9/06, A61C 19/06, A61N 1/44, A61K 33/00, A61K 6/00, A61K 47/02

(54) **Device for the topical application of medical ozone for the treatment of oral cavity diseases**
Vorrichtung für die topische Anwendung des medizinischen Ozons zur Behandlung von Erkrankungen der Mundhöhle
Dispositif pour l'application topique d'ozone médical pour traiter des maladies de la cavité buccale.

(43) Date of publication of application: 25.06.2008
(73) Proprietor: Sanipan S.r.l., 21100 Varese (IT)
(72) Inventor: Ghiringhelli, Roberto, I-21050 Clivio (Varese) (IT); Gandolfi, Renato, I-20080 Zelo Surrigone (Milano) (IT); De Bona, Daniela, I-21050 Clivio (Varese) (IT); Maniezzo, Massimo, I-24043 Caravaggio (Bergamo) (IT); Marangelli, Andrea, I-21047 Saronno (Varese) (IT)
(74) Representative: Ferreccio, Rinaldo

(56) References cited:
- WO-A-96/32901
- WO-A-99/64020
- WO-A-02/054971
- WO-A-02/078663
- WO-A-2007/046122
- DE-A- 19 932 570
- DE-A-102004 025 509
- US-A- 5 924 863
- US-A1- 2006 074 129

## Description

### Field of Application

The present invention relates to the medical use of ozone for the production of a medication for the topical treatment of oral cavity diseases.

The present invention relates, moreover, to a device, and to a system comprising such a device, for the topical application of medical ozone for the treatment of the aforesaid diseases.

### Prior Art

It is known that man is potentially subject to many oral cavity diseases.

It is also known to use ozone for the treatment of such diseases. In particular, the use of ozone is known for the treatment of caries and dental pulpitis.

Ozone (O₃), in fact, has a strong bactericidal and antiseptic ability, so that it is used, among others, in the industrial field such as for example in the area of water disinfection and surgical material sterilization and in the medical field, in oxygen mixture form, in the cure of some bacterial and viral diseases.

Because of its instability, that makes its storage difficult, ozone is generally generated at the time of need and immediately used or stored for a very short time.

In particular, in the use of ozone for the treatment of caries, ozone is generated at the same moment its use is needed by a generator that uses, as source of oxygen to transform into ozone, an ambient air flow.

In one such generator, electrical discharges resolve the diatomic oxygen molecules present in the ambient air flow into oxygen atoms that, recombining, form the triatomic ozone molecules.

The gaseous flow containing the generated ozone is then conveyed, by a distributor, to the zone to be subjected to the treatment, i.e. the tooth affected by decay.

Although advantageous, an application of this kind is not free of drawbacks, including the presence, in the gaseous mixture containing the generated ozone, of a high percentage of nitrogen oxides that are harmful and/or toxic.

The electrical discharges by which ozone is generated, in fact, also produce an activation of the diatomic nitrogen (N₂) present in the ambient air (about 78%), following which also the aforesaid undesired nitrogen oxides are generated.

It should be considered, furthermore, that various impurities are also generally present in the ambient air such as, for example, atmospheric dust and unburnt hydrocarbons, besides many other volatile compounds, which are harmful and/or toxic substances themselves and that may even, following the high voltages to which they are subjected in the ozone generator, be transformed if possible into substances which are even more dangerous for humans.

Other oral cavity diseases are instead treated with classical antibiotic therapy or with surgical therapy, but in both cases the results are not always satisfactory.

In particular, in the treatment of mandible necrosis, a standardised protocol which is able to effectively contrast the problem does not exist at present.

Although recently even for this pathology the possibility to use the ozone therapy has emerged, the results so far obtained are unsatisfactory and the surfaced drawbacks are basically of the same kind disclosed above, since also in this case ozone generated by an ambient air flow is employed.

A further disadvantage connected to the use of ozone, in particular in the treatment of oral cavity diseases, resides in the necessity to confine the ozone within the diseased zone to be subject to the treatment.

It is in fact known that ozone is a strong oxidiser, much more powerful than diatomic oxygen, and for this it results particularly toxic both by contact, with weak shown toxicity, and by inhalation with very strong shown toxicity.

In contact with cells, ozone produces an interruption of the cellular membranes, breaking them, and moreover, exercising a similar action also on the cytoplasm, causing the death of the target organism.

However, the ozone action is not selective in the sense that it is not able to distinguish between "good" organisms and "bad" organisms, so that the ability to make a precise confinement of the ozone, limited to the zone to be subject to the treatment, is of particular importance.

For the same reason, there is the necessity to make the ozone neutralization at the end of the treatment so that ozone is not dispersed into the ambient air for which, moreover, severe concentration limits are imposed by international legislations.

Therefore, mandible necrosis as with other oral cavity pathologies here considered, as well as other pathologies affecting the oral cavity, represent a substantial, unresolved problem nowadays for the health care industry, for which the prior art continues to give unsatisfactory solutions both from the prevention point of view as well as that of the real cure.

The present invention provides means for the cure or the prevention of oral cavity diseases, in particular means which overcome the abovementioned drawbacks and such that guarantee an effective treatment, intended as cure or prevention of oral cavity diseases, without counterindications for he who undergoes the treatment itself.

Document WO 02/078663 discloses an apparatus for the treatment of dental caries comprising a source of oxidizing gas and a handpiece for delivering the gas to a tooth. A cup attached to the handpiece is provided for receiving the gas and exposing a selected area of the tooth to the gas.

Document US 2006/074129 discloses a process for the obtainment of ozonized oils and fats in a bubbling reactor in which an ozonation step is performed in liquid phase of an emulsion of water in vegetable oil or fat.

Document DE 199 32 570 discloses an antimicrobial preparation releasing atomic and molecular oxygen, comprises olive oil and/or castor oil enriched with ozone.

Document WO 99/64020 relates to the use of ozone in the treatment of dental caries. The ozone is delivered at a pressure sufficient to penetrate the carious tissue in form of pure ozone or ozonized air in an aqueous medium.

Document WO 2007/046122, which is relevant to the question of novelty pursuant to article 54(3) and (4) of the EPC, discloses a process for the preparation of ozonised oil and pharmacological composition for the preparation of a medicament for the treatment of various diseases, comprising, among others, gum infections, stomatitises, aphtha, gum lesion.

Document US 5,924,863 discloses a device for delivering a predetermined treatment agent comprising a mouthguard shaped for extended contact with a substantial portion of the users mouth, such as all of the teeth and gums, so as to provide treatment to a major region of the mouth as opposed to specific topical points of treatment.

### Summary of the invention

Briefly, the present invention provides means for the topical application of medical ozone for the treatment of oral cavity diseases such as, for example, oral cavity infective and/or inflammatory lesions and oral cavity infective necrotic mucous lesions and infective necrotic osseous lesions derived, in particular but not only, from the use of bisphosphonates.

With medical ozone is meant ozone obtained from medical oxygen, that is from pure oxygen or from a mixture basically consisting in oxygen (O₂≥90%) in which the nitrogen concentration is zero or close to zero.

Within the scope of certain embodiments, the present invention refers to a device for the topical application of medical ozone for the treatment of oral cavity diseases.

According to one embodiment, such device includes an open sterilization chamber comprising an at least partial oral cavity negative mould of a subject destined to a treatment with medical ozone, in which said chamber is destined to convey, i.e. to collect and topically direct medical ozone into an oral cavity affected by pathology.

Advantageously, the aforesaid medical ozone is gaseous medical ozone released from an oily carrier such as an ozonised oil or an gel (saturated with medical ozone).

According to another embodiment, the present invention discloses a device of the aforesaid type wherein said open sterilization chamber is destined to topically convey medical ozone into, and remove it from, a zone of the oral cavity affected by pathology, wherein at said zone affected by pathology, the aforesaid mould is provided with a cavity and openings provided in said cavity for the passage of related medical ozone conveyors, respectively inlet and outlet conveyors, and wherein said mould is substantially firmly attached to an odontological impression tray crossed by said conveyors.

Advantageously, in this last case, the aforesaid medical ozone is gaseous medical ozone contained in a gaseous flow fed into said cavity.

By odontological impression tray, it is intended a conventional odontological impression tray of predetermined size, of those used to obtain, by means of suitable hardening paste, a hardened paste negative mould that at least partially reproduces the oral cavity of a subject destined for treatment with medical ozone.

The aforesaid odontological impression tray and the mould firmly attached to it represent what is here defined as an individual impression tray, as it will be better disclosed in the course of the description.

Still within the scope of other embodiments, the present invention relates to a device for the topical application of medical ozone for the treatment of oral cavity diseases, wherein said system includes the aforesaid individual impression tray for the topical application of medical ozone, a source of medical ozone connected to said individual impression tray and fed with medical oxygen (ultrafiltered) or with pure oxygen, and means to neutralise the medical ozone coming out from the aforesaid individual impression tray.

Furthermore characteristics and the advantages of this invention will be clearer from the detailed description, accompanied by several examples of use of devices for the topical application of medical ozone for the treatment of oral cavity diseases according to the invention, and disclosed hereby with reference to the enclosed drawings, provided for illustrating and non-limiting purposes.

### Brief description of the drawings

In such drawings:
Figure 1 represents a schematic frontal view of a individual impression tray used in accordance with the present invention;
Figure 2 represents a schematic rear view of the impression tray of figure 1;
Figure 3 represents a schematic side view of the impression tray of figure 1;
Figure 4 represents a functional scheme of a system comprising the individual impression tray of figure 1, for the topical application of medical ozone for the treatment of oral cavity diseases according to the present invention;
Figure 5 represents a functional scheme of a variant embodiment of the system of figure 4.

### Detailed description

The present invention showed the particular effectiveness and the absence of undesired effects derived from the use of medical ozone in the production of a medication for the treatment of oral cavity diseases such as infective and/or inflammatory lesions of the oral cavity and the infective necrotic mucous lesions and infective necrotic osseous lesions, deriving from the use of bisphosphonates among other substances.

The aforesaid medication was made by saturating an oily carrier with medical ozone, such as an oil or a gel, with the obtainment of an ozonised oil.

In particular, a vegetable oil saturated with medical ozone has been obtained according to the following embodiments.

From olive oil samples containing:
70-80% of oleic acid (unsaturated)
4-12% of linoleic acid (unsaturated)
7-15% of palmitic acid (saturated)
2-6% of stearic oil (saturated)
and from sunflower seed oil samples containing about 64% polyunsaturated fatty acids.

Since the higher the linoleic acid (unsaturated fat) concentration, the more unstable the oil is to oxidation, and since saturation with ozone follows an oxidation mechanism, it was necessary to have oils with the lowest possible linoleic acid content.

In this way the ozone-oil bond (that is the bond between ozone and unsaturated fatty acids) is, over time, as stable as possible.

It should be observed that the aforesaid bond is subject to resolution with temperature variation, so that the product obtained as saturated ozonised oil was stored at cool average temperatures (4-5 °C).

It should be noted moreover that in the use, that is in the treatment of an oral cavity disease, the body temperature of a subject on which the ozonised oil has been used was shown to be optimal, achieving a change of state from gel to sol, and, consequently, the oil desaturation and the liberation of medical ozone in gaseous form.

The aforesaid olive oil and sunflower seed oil samples, were saturated with medical ozone according to the procedure reported below.

An oxygen synthesiser (or oxygen concentrator), equipped with a flowmeter and lacking a gas humidifier, has been connected to an ozone generator.

In particular, a synthesiser able to synthesise 98% of the atmospheric oxygen present in an ambient air gas flow fed to the synthesiser has been employed.

During its passage through the oxygen synthesiser, the ambient air gas flow fed therein has moreover been ultrafiltered.

In this manner, it has been possible to have a nearly inexhaustible source of almost pure oxygen (medical oxygen), with a near zero reduction of the nitrogen concentration and with nearly total removal of the peculiar impurities of the atmospheric air.

Consequently, the probability that, in the course of its path, the obtained gas flow (medical oxygen) contained nitrogen oxides, in particular nitrogen peroxide, or harmful air-dispersed substances was close to zero.

The pure oxygen flow (medical oxygen flow), adjusted to 0.15-0.2 pressure bars, has then been pushed by the synthesiser to cross through the aforesaid ozone generator capable of producing 4/5 g/h of medical ozone.

The ozone generator is basically composed by an electrical apparatus comprising electrodes in which, by means of a transformer that brings the line voltage from 220V to very high values, a high potential zone is generated.

In this manner, a series of electrical discharges between the electrodes are caused to be released, and the oxygen flow that passes through the high potential electrical arc is converted into a flow of O₃ (ozone).

The ozone concentration is inversely proportional, generator power being equal, to the volume of oxygen passed through the generator itself, so that, by adjusting the flowmeter of the oxygen synthesiser upstream of the system it was possible to control the medical ozone concentration at the generator outlet.

In practice, the flowmeter permits regulating the oxygen flow that, pushed by the compression system of the synthesiser, is directed to the medical ozone generator.

In this manner it was possible to have a nearly inexhaustible source of medical oxygen and therefore of medical ozone, which, pushed by the same synthesiser compression forces that cross the ozone generator, was driven into the oil.

In particular, the oil was poured in a sealed container, on which bottom a gas diffuser was anchored.

The gas diffuser was connected to the ozone generator by an inlet duct whose inlet into the container was carefully sealed to avoid gas dispersions into the environment.

The oil container was connected, by an ozone outlet duct placed over the oil level, to an ozone neutralisation filter containing "Carulite" and hermetically sealed, provided with an opening downstream of the filter which allowed the dispersion into the atmosphere of the ozone reconverted in oxygen by the filter itself.

Also the aforesaid ozone inlet duct was carefully sealed to avoid environmental dispersions of gas.

The passage of the ozone through the diffuser, immersed in the vegetable oil, allowed its saturation, which occurred in about 28 days under continuous infusion (24 hours a day).

The result was a saturated gel of medical ozone, suitable for the topical application for human use in the treatment of oral cavity diseases as here described.

The saturation, as mentioned above, was maintained by the low temperatures (4-5 °C), whereas the medical ozone liberation happened during the transition of the carrier from gel to sol.

The body temperature (37 °C) in a subject on which the oil has been used appeared ideal for the aforesaid transition.

In the infective and/or inflammatory pathologies of the oral cavity and on the osseous and mucous lesions of the mouth from bisphosphonates, the use procedure of the ozonised oil according to the invention was the following.

A negative mould of the superior and inferior dental rimae of a treated subject (patient) has been manufactured with a common odontological (dental) impression tray of predetermined suitable measure.

The mould was obtained by spreading the dental impression paste as much as possible beyond the gingival junction margin.

The hardened mould was then separated from the impression tray and gel to be used was spread over; the mould became the gel's container, here also defined as open sterilization chamber.

To spread the gel over the mould, a brush was used but a spatula was also suitable for the operation.

The gel can be spread over the mould on the whole dental rima or only a portion at a localised oral cavity lesion.

The negative mould was then repositioned in the treated subject's mouth and therein left for about 10 minutes, after which a surgical aspirator was placed in the subject's oral cavity to remove possible ozone leaks from the sterilization chamber (mould), since the gas cannot be inhaled.

The gaseous medical ozone, with which the gel was permeated, was released because of the heating of the gel itself in contact with the human tissues.

It is known that ozone has oxidising and biocidal abilities, favouring neoangiogenesis and neofibroblastogenesis and, therefore, is disinfectant and cicatrising.

Moreover, following the use of medical ozone for the production of a medication for the treatment of oral cavity diseases according to the present invention, it has been possible to verify:
- in a sample of ten subjects affected by periodontal disease (pyorrhoea) in an acute phase, treated with such medication according to the aforesaid procedure with applications of the duration of ten minutes each, repeated twice a week for five weeks, a notable, that is, a very pronounced, surprising regression of the symptoms (bleeding, pain, cleansing of the purulent gingival pouches etc.), and a gingival retraction stasis in all of the treated subjects.

The test was confirmed by a medical examination at a distance of three months from the last application.
- in a sample of four subjects affected by infected post-extractive lesions, treated with such medication according to the aforesaid procedure with applications of the duration of ten minutes each, repeated with a two day interval for overall eight days, a complete regression of the infective focus.
- in a sample of seven subjects affected by infective and/or inflammatory gingival diseases caused by stress, hepatosis, vitamin deficiency or more, treated with such medication according to the aforesaid procedure with applications of the duration of ten minutes each, repeated with a two day interval for overall ten days, a complete recovery of the clinical picture (gingival bleeding, halitosis, foetus ex ore, sialorrhea etc.).

In accordance with the present invention the effectiveness was then verified of the medication as described above in the treatment of oral cavity diseases such as lesions from bisphosphonates.

Briefly, the bisphosphonates are drugs that act on bone structure and on organism mineralisation.

They are indicated in the treatment of osteoporosis, especially post-menopause and, reducing the risk of fractures, as supporting the maintenance of skeletal integrity in specific oncological and other treatments.

It has been verified that these drugs , in addition to the side effects described in the literature, cause, in some cases, the osteonecrosis of upper and lower maxillary bones and the necrosis of the oral cavity mucosae with an inhibition mechanism of the neoangiogenesis.

This causes, from a practical point of view, the enlargement of the post-extractive lesions, until osseous perforation, in subjects under treatment with bisphosphonates.

According to the applicant, a hypothesis on the causality of the generator event of the aforesaid lesions could be the possibility that the bisphosphonates, entered into the human organism, produce a harmful metabolite.

The reason why these lesions are found only in the oral cavity might be attributed to the fact that this metabolite might reach, in and only in the mouth, higher concentrations than in the other zones, such to overcome the organism's buffer systems.

The concentrations are so high because the harmful metabolite might be brought here either from the blood stream or the salivary excretion, so that this pincer mechanism might cause the pathology.

The literature moreover describes side effects in the use of drugs based on bisphosphonates at an esophageal or gastric level whose aetiology might be referred to the acidity caused by the drug.

It could be then hypothesised that the destroying action from bisphosphonates in the oral cavity might be imputable to the formation of an acid and necrotising compound.

These characteristics are similar to those induced by the contact with phosphoric acid.

In any case, either the harmful metabolite is carried into the oral cavity by blood and saliva, or alternatively, it is formed in situ by contact with enzymes and aqueous salivary solution, it was verified as a fact that the use of the ozonised oil according to the present invention showed neutralising ability, which justifies the medical ozone therapeutic action on lesions of the aforesaid type.

This ability might be due to the oxidative mechanism of the medical ozone released in the gaseous state from the oily carrier, which combines with the free radicals of the acid, forming a chelated acid, transforming it into an amorphous compound.

In particular, in two subjects affected by post-extractive pathologies induced by bisphosphonates as considered above, treated with medical ozone saturated oil according to the invention, at a distance of twelve months from the extraction, when the infective necrotic lesions were in an acute phase, administered according to the above described procedure, it was verified:
- after the first application of the ozonised oil, the almost complete regression of the infective pathology and the inflammatory symptoms (acuteness).

It was thereof possible to proceed, afterwards, to the cleansing of the post-extractive wound, with a dimension of 2 cm x 3 cm (more or less with identical dimensions in both treated cases), with a diamond tip assembled on a subsonic apparatus (40 nm of vibratory range) without the use of local anaesthesia.

It was verified, before the treatment with the oil according to the present invention, that the mucous wound edges, after one year from the extraction, showed atrophic and hypoemic edges and the bone was brown-coloured.

It was further verified, at the time of the tenth oil application as above disclosed, that the gingival edges were hyperemic, swollen, and overlapping the preceding lesion edges (starting of the healing by second intention) and the bone returned light-coloured, even if avascular.

This could likely be attributed to the neutralisation of the acid, as previously hypothesised, with which the bone could have been soaked and to the consequent tissue reepithelisation.

It was further verified that after the twentieth oil application as above disclosed, the wound was reduced of 2/3 the starting dimensions with exposure of only the post-extractive acetabular recess.

The complete disappearance was moreover verified of the painful symptomatology, even from the dental elements facing the lesion.

The bone was remineralised, as in the control radiography, the edges of the residue wound were shown to be hyperemic and the cicatricial mucous coating the most of the bone.

The previous sick zone had a very normal rose-colored appearance, and cicatricial retractions were not evident.

In practice, considering the clean positive change of the clinical picture in the subjects treated according to what above described, with almost total regression of the oral cavity diseases induced by bisphosphonates, the possibility to successfully conclude a similar treatment should be seriously taken into consideration.

These encouraging results urge the use of oil saturated with medical ozone for the treatment of oral cavity diseases, in particular for diseases of the aforesaid kind induced by bisphosphonates.

For the latter diseases, the wound recovery could occur due to the biocidal action of the medical ozone that does not allow the overlapping of infections causing a chronic atrophic process and due to the chelating action of the medical ozone, which neutralises the burning necrotising compound, and due to the direct action of the medical ozone on the mechanisms of vascular neoformation and cicatrisation by means of neofibroblastogenesis.

The foregoing is supported by the fact that the recovery was verified of a lesion formed one year before the beginning of the treatment by means of oil saturated with medical ozone in accordance with the invention, which was expanded during the period between its formation and the beginning of the administration of the oil saturated with medical ozone, and was then completely regressed in the space of six months in which the therapy with the aforesaid oil took place.

Therefore, in accordance with the foregoing, the present invention provides a device including an open sterilization chamber comprising an at least partial negative mould of the oral cavity of a subject for use in the treatment with medical ozone, in which said chamber is intended to convey, i.e. to collect and topically direct medical ozone into a oral cavity zone affected by the pathology.

In particular, medical ozone is released in the gaseous state from a oily carrier such as an oil or a gel saturated with medical ozone.

The possibility was verified to further improve the above described results by the direct use of gaseous medical ozone.

In particular, it was desired to verify the effectiveness of the treatment with medical ozone even in case of lesions from bisphosphonates more serious than the ones considered above, having a diameter equal or greater than three centimetres.

For the purpose, the present invention provides an open sterilisation chamber of the aforesaid type, intended to topically convey medical ozone into, and to remove it from, a zone of the oral cavity affected by pathology, wherein, at the zone affected by pathology, the aforesaid mould is provided with a cavity and openings provided in said cavity for the passage of related respectively inlet and outlet conveyors of medical ozone, and in which the mould is substantially firmly attached to an odontological impression tray crossed by the aforesaid conveyors.

In this case, as medical ozone source, a gaseous flow comprising medical ozone is used which is fed by the aforesaid inlet conveyor into the aforesaid cavity.

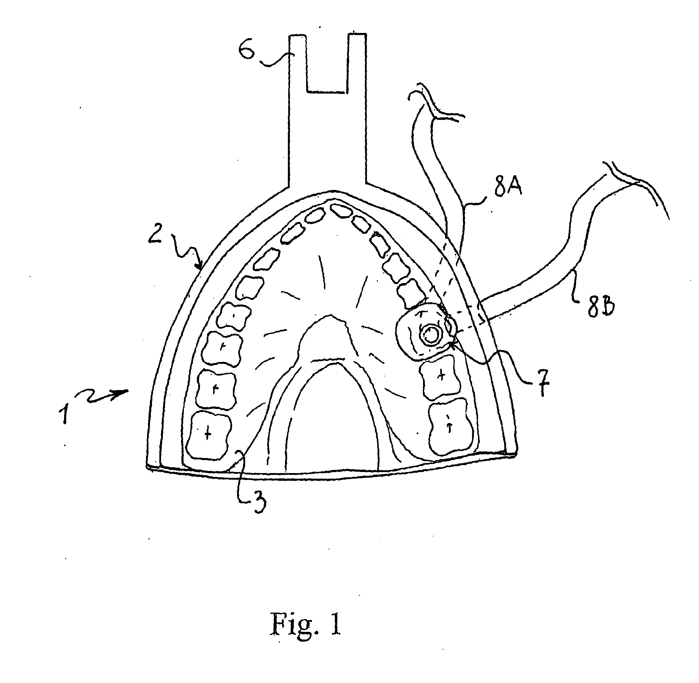

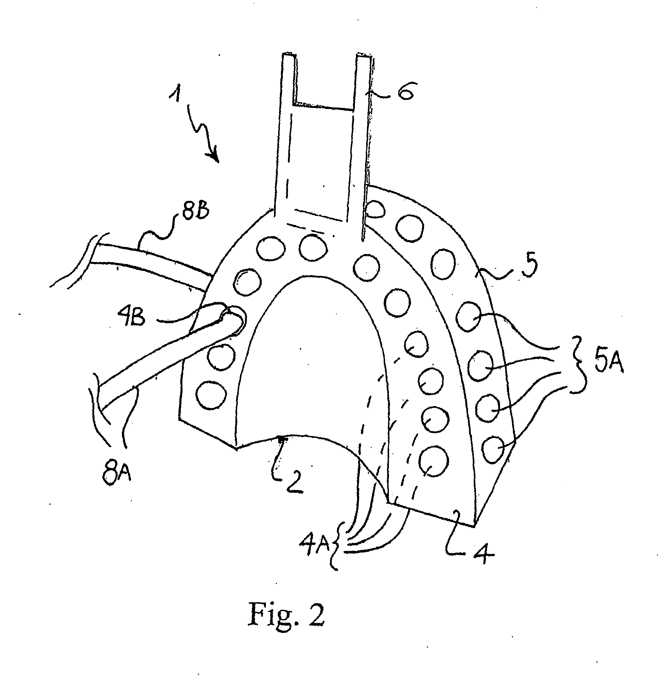

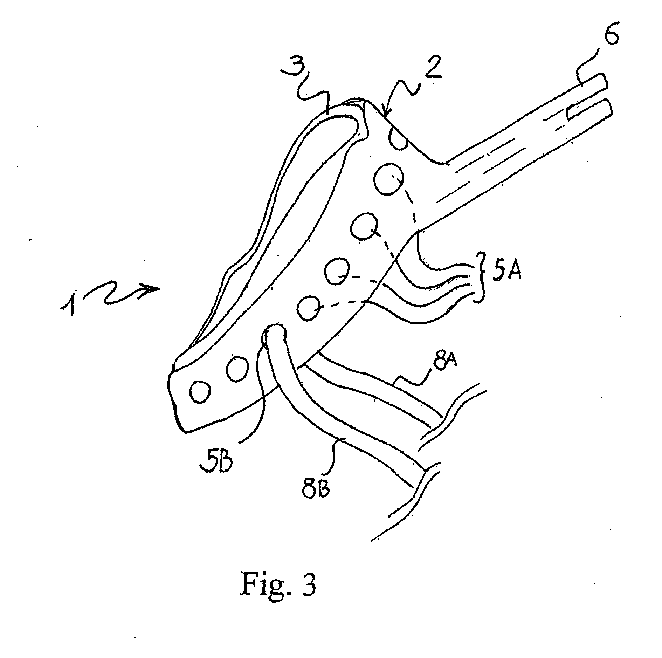

Within the scope of the present invention, the mould and the odontological impression tray together constitute an individual impression tray, which is hereafter illustrated with reference to figures 1-3 where it is indicated in its entirety as 1.

Therefore, the individual impression tray 1 comprises essentially a odontological impression tray 2 of known type, for example made of steel, to which an at least partial negative mould 3 of the oral cavity of a subject destined for a treatment with medical ozone is substantially firmly attached.

In detail, the impression tray 2 comprises a substantially solid half-elliptical shaped first portion 4, and a second U-shaped portion 5 (empty half-ellipse shape) which is arranged substantially in an orthogonal manner to the first portion 4, forming a raised edge for the latter.

At a median portion of the impression tray 2 there is a projecting grip 6 which allows the easy handling of the individual impression tray 1.

The aforesaid first portion 4 of the impression tray 2 constitutes the base of the impression tray itself and it is intended, upon the interposition of a hardening paste to obtain the mould 3, to abut against the superior or inferior wall of a subject's oral cavity.

The second portion 5 of the impression tray 2 is instead intended to abut against the frontal wall of a subject's teeth.

Both the aforesaid portions, first 4 and second 5, have a series of full thickness holes, 4A and 5A respectively, arranged in a substantially corresponding manner.

In practice, in the first portion 4 of the impression tray 2, the first series of holes 4A is arranged at the U-shaped portion of the half-elliptical perimeter edge.

In a corresponding manner, the second series of holes 5A is arranged in the second portion 5 of the impression tray 2.

As mentioned above, firmly attached on the impression tray 2, the mould 3 is made from a suitable hardening paste, generally a silicon-based paste.

The mould 3 exactly reports imprinted the impression of at least a part of the oral cavity of a subject affected by a pathology of the type previously considered, apart a predetermined portion corresponding with a zone of the oral cavity affected by the pathology, which is made hollow to form a cavity 7, here defined also as bell-shaped section.

It should be noted that the mould 3 obstructs the holes of the two series of holes 4A, 5A of the impression tray 2, so to seal them, except for two holes, one hole per series, placed at the cavity 6 and respectively indicated with 4B and 5B.

The aforesaid holes 4B, 5B, constitute therefore respective openings for the passage of related conveyors 8A and 8B, one inlet and one outlet, of a gaseous flow comprising medical ozone to convey into the zone affected by pathology.

In practice, in the individual impression tray 1, a conveyor is inserted bringing medical ozone, for example the conveyor 8A, which penetrates and crosses its structure engaging related and corresponding openings of the impression tray 2 and the mould 3, and a conveyor flows away medical ozone, in the example of the figure the conveyor 8B, which in a similar way penetrates and crosses the individual impression tray 1.

The conveyors 8A and 8B are then arranged in the individual impression tray 1 at the cavity or bell-shaped section 6, obtained by excavation into the mould 3, which as previously described is provided in a portion of the mould 3 corresponding to the zone affected by pathology here considered.

The cavity 6 is dedicated to the containing of the gas which, in the use of the individual impression tray 1, is introduced in the cavity 6 by means of the inlet conveyor of the gaseous flow comprising medical ozone.

The cavity 6, always in the use of the individual impression tray 1, isolates the zone affected by pathology, allowing the action thereon of the medical ozone there convoyed.

The outlet conveyor of the gaseous flow comprising medical ozone, always placed at the cavity 6, allows instead the outflow of the gas introduced without causing its dispersion in the air.

In detail, to feed the gaseous flow comprising medical ozone as above reported into the cavity 6, the individual impression tray 1 is connected by the inlet conveyor 8A to a medical ozone generator of the type above disclosed which is controlled, in all the functions, by a programmable PLC to be able to manage, with the maximal precision, the medical ozone administration times and possible safety systems connected to it.

In particular, it is provided a system that comprises, in series, an oxygen synthesiser of the type above considered for the synthesis of medical oxygen from ambient air, the aforesaid medical ozone generator by means of which the medical oxygen flow is converted in medical ozone and which, as mentioned above, is connected to the individual impression tray by means of the inlet conveyor of the gaseous flow comprising medical ozone.

Downstream of the outlet conveyor, means are instead provided to neutralise the medical ozone present in the gaseous flow which leaves the sterilisation chamber.

Such neutralisation means preferably consist of a hermetically-sealed filter provided with a catalyst, for example Carulite granules.

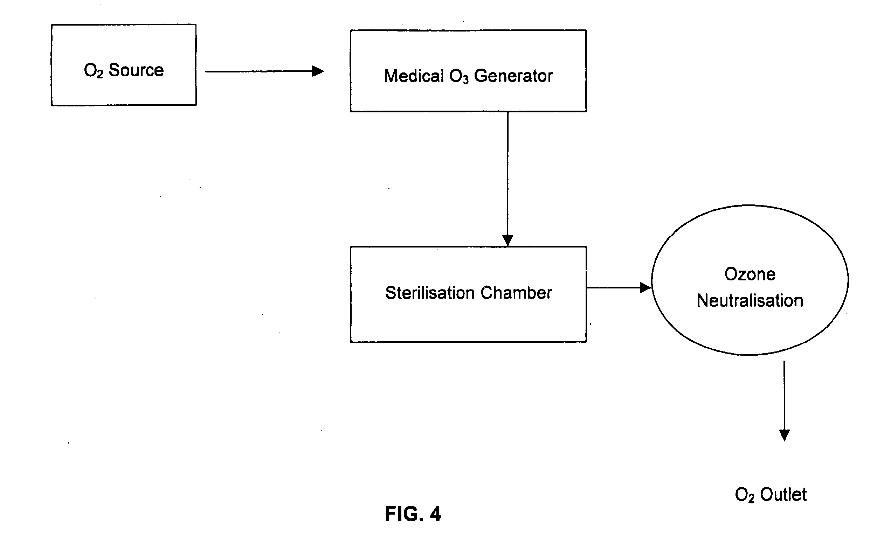

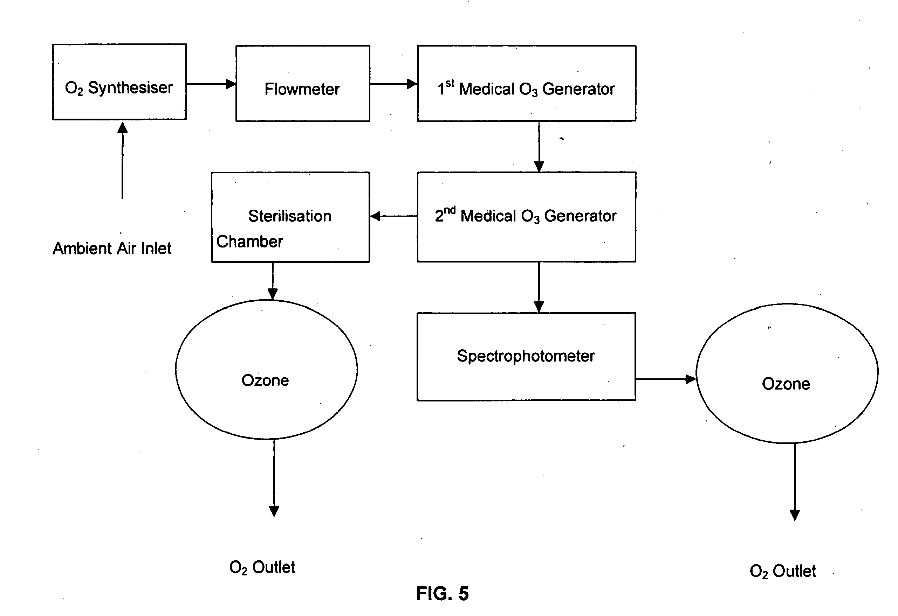

The catalyst reconverts the medical ozone into oxygen (diatomic oxygen), avoiding an undesired and harmful ozone introduction into the atmosphere, as it is represented in the example of figure 4 and 5.

Preferably, between the oxygen synthesiser and the ozone generator, a flowmeter is interposed which controls the medical oxygen flow, also of the above-considered type, whereas according to another embodiment the medical ozone generator is also connected, downstream, to an ozone spectrophotometer.

The ozone spectrophotometer, in which a portion of the gaseous flow comprising medical ozone is sent, is provided with a display for signalling the medical ozone concentration rate, calibrated into a range of 0-128 µg x ml.

In such case, also downstream of the ozone spectrophotometer, neutralisation means are provided , for example of the same above considered type, to reconvert the medical ozone into oxygen.

It should be said that to generate medical ozone, several medical ozone generators can be foreseen arranged in series, the last of which will be connected to the spectrophotometer and to the sterilisation chamber, as is illustrated in the example of figure 5, in which a first and a second medical ozone generator are represented.

It should said moreover be said that, instead of the aforesaid oxygen synthesiser, a medical or pure oxygen cylinder can be used as source of medical oxygen.

In an alternative embodiment, the system according to the invention for the use of the individual impression tray also comprises suction means preferably comprising a siphon and a suction pump, respectively placed upstream and downstream of the filter connected to the outlet conveyor (first filter), as represented in the example of figure 5.

In this way the outlet conveyor results connected to the siphon, which is connected to the first filter which is itself connected to the suction pump.

The suction pump, when in function, has the purpose of producing a slight depression inside the cavity (bell-shaped section) of the mould, so that the outflow of the gaseous flow comprising medical ozone from the sterilization chamber is further assisted, having therefore the certainty that incidental medical ozone dispersions cannot be introduced in the patient's oral cavity.

For the realisation of the pneumatic connections between the above disclosed system components are preferably used flexible tubes in Teflon®, stainless steel pipe fittings, Viton® gaskets, or in any case other materials which show a strong ozone resistance.

The aforesaid disposition on the whole achieves a system that allows the topical and continuous application of medical ozone for the treatment of oral cavity diseases with a total control in real time of the medical ozone concentration that is administered thanks to the use of the individual impression tray previously disclosed.

Essentially, in the use, what is following happens: the gaseous flow comprising medical ozone is conveyed with a certain pressure, by the inlet conveyor, into the sterilization chamber and here, confined by the mould cavity, the medical ozone in contact with the zone affected by pathology performs its healing action.

The gaseous flow under pressure, in the mould cavity, finds then the outlet conveyor as obligatory exit, through which the medical ozone leaves the sterilisation chamber without there being any further contact with other oral cavity zones.

If needed and once the therapeutic action is completed, the possible siphon and the suction pump further favour the removal of the medical ozone from the sterilisation chamber.

The advantages of the present invention, already evident in the above reported description, essentially consist in the fact that the invention provides means for the cure or the prevention of oral cavity diseases such to ensure an effective treatment of such diseases without the manifestation of counterindications or undesired effects for the subject who undergoes the treatment itself.

Essentially the present invention provided means for the topical application of the medical ozone for the treatment of oral cavity diseases such as infective and/or inflammatory lesions of the oral cavity, for example abscesses and the like, and infective necrotic mucous lesions and infective necrotic osseous lesions of the oral cavity, deriving in particular but not only from the use of bisphosphonates (necrosis of chemical origin).

The present invention achieves the aforesaid advantages by virtue of the use of medical ozone for the production of a medication for the treatment of diseases of the aforesaid type, by virtue of a device used for the application of such medication, by virtue of a device used for the continuous application of gaseous medical ozone for the treatment of oral cavity diseases and a system comprising such device.

The present invention allows employing gaseous medical ozone in a continuous manner for the treatment of oral cavity diseases in an effective and safe way.

Advantageously, the present invention provided medical ozone in a continuous way and it provides a practically inexhaustible source of medical ozone.

The fact that the gaseous flow comprising medical ozone is a continuous flow fed to the sterilisation chamber ensures a constant medical ozone concentration at the oral cavity zone affected by the pathology to be treated.

This is of primary importance since the hardening paste by which the mould is made is generally a silicon paste that tends to absorb ozone, and that therefore in use it would cause to an undesired progressive diminution of the medical ozone in the zone to be treated.

The use of the present invention moreover allows to flow medical ozone through the sterilisation chamber as described above in its different embodiments, only and exclusively in the zone affected by one or more of the aforesaid oral cavity diseases, avoiding contacts with healthy zones, to which it might however cause damage.

Due to the confinement of the gaseous flow comprising medical ozone and to the possibility that such gaseous flow has to leave the sterilisation chamber, the medical ozone inhalation which is extremely harmful for the human organism is moreover avoided.

By virtue of the foregoing, even the silicon compounds that might be generated from the mould degradation caused by the medical ozone, potentially harmful and/or toxic for man, are removed from the sterilization chamber and thereof from the oral cavity.

Inhalations and contact with ozone are moreover avoided due to the fact that the medical ozone employed is always neutralised (reconverted into diatomic oxygen) at the end of the use, and that is before that the gaseous flow is released in the atmosphere.

A further advantage consists in the real time monitoring of the produced and employed medical ozone, which is easily and effectively attained.

## Claims

1. System for continuous topical application of medical ozone for the treatment of oral cavity diseases comprising, connected in series with each other:
a source of medical oxygen (ultrafiltered oxygen) or pure oxygen;
at least one medical ozone generator to transform said medical oxygen into gaseous medical ozone;
a device to topically apply said gaseous medical ozone to a zone of the oral cavity affected by pathology, wherein said device includes an open sterilisation chamber comprising an at least partial negative mould of the oral cavity of a subject destined for a treatment with medical ozone and an odontological impression tray on which said mould is firmly attached, wherein said chamber is destined to topically convey medical ozone into, and remove it from, a zone of the oral cavity affected by pathology, wherein at said zone affected by pathology said mould is provided with a cavity and openings arranged in said cavity for the passage of related medical ozone conveyors, respectively inlet and outlet conveyors, crossing said odontological impression tray;
means for neutralising the medical ozone exiting from said sterilisation chamber.

2. System according to claim 1, further comprising suction means including a siphon and a suction pump, respectively placed upstream and downstream of said means of neutralization of the medical ozone.

3. System according to claim 1 or 2, further comprising a flowmeter interposed between said medical oxygen source and said at least one medical ozone generator.

4. System according to any one of the claims 1-3, further comprising an ozone spectrophotometer connected downstream of said at least one medical ozone generator to receive a portion of the gaseous flow comprising medical ozone.

5. System according to claim 4, further comprising means for neutralising the medical ozone exiting from said ozone spectrophotometer.

6. System according to any one of the claims 1-5, wherein said means for neutralising the medical ozone comprise a hermetically-sealed filter provided with a catalyst.

7. System according to any one of the claims 1-6, wherein two medical ozone generators are foreseen arranged in series with each other.

8. System according to any one of the claims 1-7, wherein said medical oxygen source is composed of a synthesiser of oxygen fed with ultrafiltered ambient air.

9. System according to any one of the claims 1-7, wherein said medical oxygen source consists of a medical or pure oxygen cylinder.

10. System according to any one of the previous claims, wherein said diseases comprise infective lesions of the oral cavity and/or inflammatory lesions of the oral cavity, and/or infective necrotic mucous lesions of the oral cavity and/or infective necrotic osseous lesions of the oral cavity.

11. System according to claim 10, wherein said necrotic lesions are lesions induced by bisphosphonates.

## Patentansprüche

1. System zur kontinuierlichen lokalen Applikation von medizinischem Ozon für die Behandlung von Krankheiten der Mundhöhle umfassend, in Reihe miteinander verbunden:
eine Quelle von Sauerstoff medizinischer Qualität (ultrafiltriertem Sauerstoff) oder reinem Sauerstoff;
mindestens einen medizinischer Ozongenerator zur Umwandlung des Sauerstoffs medizinischer Qualität in gasförmiges medizinisches Ozon;
eine Vorrichtung zur lokalen Applikation des gasförmigen Ozons medizinischer Qualität auf eine Zone der Mundhöhle, die durch Krankheit betroffen ist, wobei die Vorrichtung eine offene Sterilisationskammer beinhaltet, die mindestens einen Teilabdruck der Mundhöhle des mit dem medizinischen Ozon zu behandelnden Subjektes und einem Zahnabdruckträger umfasst, auf welchem der Abdruck fest angeordnet ist, wobei die Kammer dazu bestimmt ist, lokal medizinisches Ozon ein- und auszubringen aus einer Zone der Mundhöhle, die durch die Krankheit beeinflusst ist, wobei der Abdruck an der durch die Krankheit beeinflussten Zone mit einer Ausnehmung und Öffnungen versehen ist, die in der Ausnehmung für die Durchleitung von medizinischen Ozonförderern, entsprechenden Ein- und Ausgangsförderern, angeordnet sind, die den Zahnabdruckträger kreuzen;
Mittel zum Neutralisieren des medizinischen Ozons, das aus der Sterilisationskammer austritt.

2. System nach Anspruch 1, ferner umfassend Saugmittel beinhaltend einen Siphon und eine Saugpumpe, die stromaufwärts beziehungsweise stromabwärts von den Mitteln zur Neutralisation des medizinischen Ozons angeordnet sind.

3. System nach Anspruch 1 oder 2, ferner umfassend einen Durchflussmesser, der zwischen der Quelle medizinischen Sauerstoffs und dem mindestens einen Generator medizinischen Ozons, angeordnet ist.

4. System nach irgendeinem der Ansprüche 1 bis 3, ferner umfassend mindestens ein Ozonspektrophotometer, das stromabwärts von dem mindestens einen Generator medizinischen Ozons angeschlossen ist, um einen Teil des medizinisches Ozon umfassenden Gasflusses zu empfangen.

5. System nach Anspruch 4, ferner umfassend ein Mittel zum Neutralisieren des medizinischen Ozons, das von dem Ozonspektrophotometer austritt.

6. System nach irgendeinem der Ansprüche 1 bis 5, in dem die Mittel zum Neutralisieren des medizinischen Ozons einen hermetisch abgedichteten Filter umfassen, der mit einem Katalysator versehen ist.

7. System nach irgendeinem der Ansprüche 1 bis 6, in dem zwei Generatoren medizinische Ozons vorgesehen sind, die miteinander in Reihe angeordnet sind.

8. System nach irgendeinem der Ansprüche 1 bis 7, in dem die Quelle medizinischen Sauerstoffs aus einem Sauerstoffgenerator besteht, der mit ultrafiltrierter Raumluft beaufschlagt wird.

9. System nach einem der Ansprüche 1 bis 7, in dem die medizinische Sauerstoffquelle aus einem Zylinder mit medizinischem oder reinem Sauerstoff besteht.

10. System nach irgendeinem der vorhergehenden Ansprüche, in dem die Krankheit infektiöse Wunden der Mundhöhle und/oder entzündete Wunden der Mundhöhle und/oder infektiöse, nekrotische, muköse Wunden der Mundhöhle und/oder infektiöse, nekrotische Knochenwunden der Mundhöhle umfasst.

11. System nach Anspruch 10, in dem die nekrotischen Wunden solche sind, die durch Bisphosphonate hervorgerufen sind.

## Revendications

1. Système pour l'application topique continue d'ozone médical pour le traitement de maladies de la cavité buccale comprenant, reliés en série les uns avec les vautres :
une source d'oxygène médical (oxygène ultrafiltré) ou d'oxygène pur ;
au moins un générateur d'ozone médical pour transformer ledit oxygène médical en ozone médical gazeux ;
un dispositif pour appliquer topiquement ledit ozone médical gazeux sur une zone de la cavité buccale affectée par la pathologie, dans lequel ledit dispositif comprend une chambre de stérilisation ouverte comprenant un moule négatif au moins partiel de la cavité buccale d'un sujet devant recevoir un traitement avec de l'ozone médical, et un porte-empreinte odontologique sur lequel ledit moule est solidement fixé, dans lequel ladite chambre est destinée à transporter topiquement l'ozone médical, et l'en retirer, dans une zone de la cavité buccale affectée par la pathologie, dans lequel au niveau de ladite zone affectée par la pathologie, ledit moule est muni d'une cavité et d'ouvertures agencées dans ladite cavité pour le passage de convoyeurs d'ozone médical associés, respectivement les convoyeurs d'entrée et de sortie, traversant ledit porte-empreinte odontologique ;
des moyens pour neutraliser l'ozone médical sortant de ladite chambre de stérilisation.

2. Système selon la revendication 1, comprenant en outre des moyens d'aspiration comprenant un siphon et une pompe aspirante, placés respectivement en amont et en aval desdits moyens de neutralisation de l'ozone médical.

3. Système selon la revendication 1 ou 2, comprenant en outre un débitmètre interposé entre ladite source d'oxygène médical et ledit au moins un générateur d'ozone médical.

4. Système selon l'une quelconque des revendications 1 à 3, comprenant en outre un spectrophotomètre d'ozone raccordé en aval dudit au moins un générateur d'ozone médical pour recevoir une partie de l'écoulement gazeux comprenant l'ozone médical.

5. Système selon la revendication 4, comprenant en outre des moyens pour neutraliser l'ozone médical sortant dudit spectrophotomètre d'ozone.

6. Système selon l'une quelconque des revendications 1 à 5, dans lequel lesdits moyens pour neutraliser l'ozone médical comprennent un filtre hermétiquement fermé muni d'un catalyseur.

7. Système selon l'une quelconque des revendications 1 à 6, dans lequel deux générateurs d'ozone médical sont prévus, agencés en série l'un avec l'autre.

8. Système selon l'une quelconque des revendications 1 à 7, dans lequel ladite source d'oxygène médical est composée d'un synthétiseur d'oxygène alimenté avec de l'air ambiant ultrafiltré.

9. Système selon l'une quelconque des revendications 1 à 7, dans lequel ladite source d'oxygène médical est composée d'une bouteille d'oxygène médical ou pur.

10. Système selon l'une quelconque des revendications précédentes, dans lequel lesdites maladies comprennent des lésions infectieuses de la cavité buccale et/ou des lésions inflammatoires de la cavité buccale et/ou des lésions muqueuses nécrotiques infectieuses de la cavité buccale et/ou des lésions osseuses nécrotiques infectieuses de la cavité buccale.

11. Système selon la revendication 10, dans lequel lesdites lésions sont des lésions induites par les biphosphonates.
